# EUROPEAN PATENT SPECIFICATION

(11) **EP 0 799 196 B1**
(45) Date of publication and mention of the grant of the patent: **28.07.1999**
(21) Application number: 95940263.7
(22) Date of filing: 28.11.1995
(51) Int. Cl.: C07C 309/18, C07C 303/32, C11D 3/34

(54) **DIAMINOALKYL DI(SULPHOSUCCINATES) AND DETERGENT COMPOSITIONS CONTAINING THEM**
DIAMINOALKYLDI(SULFOSUCCINATE) UND DIESE ENTHALTENDE WASCHMITTELZUSAMMENSETZUNGEN
DIAMINOALKYLE DI(SULFOSUCCINATES) ET COMPOSITIONS DETERGENTES LES CONTENANT

(30) Priority: 22.12.1994 US 362358
(43) Date of publication of application: 08.10.1997
(73) Proprietor: UNILEVER PLC, London EC4P 4BQ (GB); UNILEVER N.V., 3013 AL Rotterdam (NL)
(72) Inventor: GUTIERREZ, Eddie, Nelson, Midland Park, NJ 07432 (US); WU, Shang-Ren, Mahwah, NJ 07430 (US)
(74) Representative: Fransella, Mary Evelyn
(86) International application number: PCT/EP95/04725
(87) International publication number: WO 96/19445

(56) References cited:
- US-A- 3 957 775
- US-A- 4 704 233
- US-A- 5 258 141

## Description

### TECHNICAL AREA

The present invention relates to novel diaminoalkyl di(sulphosuccinate) (DDSS) compounds and their use as chelants for metal ions, and as builders in detergent compositions.

### BACKGROUND AND PRIOR ART

The use of aminopolycarboxylates as laundry detergent additives is generally disclosed in the art. For example, the prior art describes laundry detergent compositions which include nitrilotriacetates (NTA), ethylendediaminetetraacetates (EDTA), diethylenetriaminepentaacetates (DTPA), and hydroxyethylethylenediaminetriaacetates (HEDTA), and triethylenetetramine hexaacetic acid (TTHA).

US 4 560 492 (Procter & Gamble) discloses laundry detergent compositions containing an aluminosilicate or organic detergency builder and from about 0.5% to about 10% by weight of HEDTA. The list of suitable organic detergency builders disclosed includes aminopolycarboxylates such as NTA, EDTA and DTPA.

US 4 397 776 and EP 60 710B (Procter & Gamble) disclose liquid laundry detergent compositions having a pH between 9 and 13, containing alpha-amine oxide surfactants and from about 0.01% to about 25% by weight of a heavy-metal chelating agent. The preferred chelating agents include aminopolycarboxylates such as NTA, EDTA, DTPA and HEDTA.

US 3 920 564 (Colgate-Palmolive) discloses softener/detergent formulations containing surfactants, quaternary ammonium or diamine fabric softeners, and a builder salt selected from aminopolycarboxylates and/or sodium citrate. Examples of suitable aminopolycarboxylates include NTA, EDTA and HEDTA.

US 3 151 084 (Swift & Co/Rogers et al) discloses alkylbenzenesulphonate-containing detergent compositions in which solubility is said to be improved by the addition of a mixture of EDTA and a solubilizing agent selected from salts of N,N-di(2-hydroxyethyl) glycine, iminodiacetic acid, NTA and HEDTA.

US 4 704 233 and EP 267 653B (Procter & Gamble/ Hartmann et al) disclose detergent compositions containing ethylenediamine N-N'disuccinic acid and salts.

None of these patents or applications disclose either DDSS and its salts or detergent compositions containing it. Moreover, the aminopolycarboxylates disclosed in those patents or applications are not biodegradable.

US 4 701 284 and EP 196 596B (Bayer AG/Hendricks et al) disclose branched sulphosuccinamic acid emulsifiers which can contain two sulphonate groups and two carboxylic acid groups, but which differ from the compounds of the present invention in that they also contain an amide group.

US 5 258 141 and EP 516 102B (Dow Chemical Co./Crump et al) disclose biodegradable chelants containing sulphonate groups, but the compounds disclosed, unlike those of the present invention, do not contain two amino nitrogen atoms in the molecule.

The art also discloses methods of synthesising mono aminoalkyl sulphosuccinates and mono iminoalkyl sulphosuccinates, in US 3 957 775 (Lever Brothers Co/Lamberti).

None of these references discloses the DDSS compounds of the present invention, nor recognises the unique builder properties of DDSS in the context of laundry detergent compositions.

### DEFINITION OF THE INVENTION

The novel compounds of the present invention are generally of the formula I: 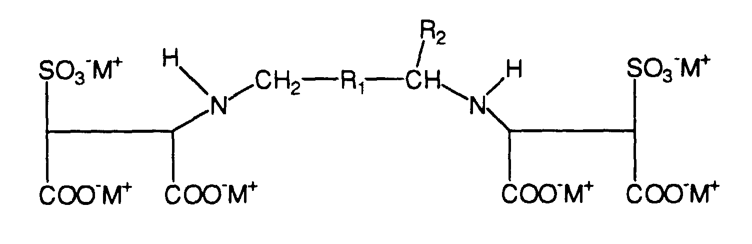 wherein
M is an alkali metal or an alkaline earth metal or a mixture; and
R₁ is CH₂ or CHOH and is optionally present, and when R₁ is present R₂ is hydrogen, and when R₁ is absent R₂ is methyl or hydrogen.

The invention further provides a process for the preparation of a compound as defined in the previous paragraph, which comprises:
(i) reacting sulphur trioxide with maleic anhydride to form sulphomaleic anhydride,
(ii)converting the sulphomaleic anhydride thus formed to sulphomaleic acid with reaction with water in the absence of alkali metal or alkaline earth metal ions,
(iii) reacting the sulphomaleic acid with a diamine selected from ethylene diamine, 1,3-propylene diamine,2-hydroxy-1,3-propylene diamine and 1,2-propylene diamine in the presence of alkali and/or alkaline earth metal hydroxide.

The invention further provides a method for chelating di- or polyvalent metal ions, especially alkaline earth metal ions, which comprises contacting said ions with a diaminoalkyl di(sulphosuccinate) compound as defined above.

The invention further provides a built detergent composition which comprises one or more detergent surfactants and, as a detergency builder, from 0.1 to 75 wt% of a diaminoalkyl di(sulphosuccinate) compound as defined above.

### DETAILED DESCRIPTION OF THE INVENTION

### Preferred compounds

In the compounds of the invention, which have the general formula I, the group R₁, which represents CH₂ or CHOH, may be present or absent. When R₁ is present R₂ is hydrogen, and when R₁ is absent R₂ is methyl or hydrogen.

In preferred compounds of the invention,
- R₁ is absent and M is sodium; or
- R₁ is absent, R₂ is methyl and M is sodium; or
- R₁ is CH₂ and M is sodium; or
- R₁ is CHOH and M is sodium.

Preferably R₁ is not present and R₂ is hydrogen so that the two sulphosuccinate groups are connected by ethylenediamine.

The compounds of the invention include the alkali metal or alkaline earth metal salts of ethylenediamine di(sulphosuccinate), 1,3-propylenediamine di (sulphosuccinate) 1,2-propylenediamine di(sulphosuccinate), and 2-hydroxy-1,3-propylenediamine di(sulphosuccinate).

Especially preferred compounds of the invention are: hexasodium ethylenediamine di(sulphosuccinate); hexasodium 1,3-propylenediamine di(sulphosuccinate); and hexasodium 2-hydroxy-1,3-propylenediamine di(sulphosuccinate).

### Preparation of the compounds

A workable and cost-efficient production of DDSS and its salts must be directed towards optimising the process conditions in such a manner that reasonable yields are obtained.

The DDSS may suitably be prepared by the following process:
(i) reaction of sulphur trioxide with maleic anhydride, preferably at a mole ratio of the sulphur trioxide to maleic anhydride of from 1.1:1 to 1.3:1 and preferably at a temperature of 60°C to 80°C, to form sulphomaleic anhydride;
(ii) addition of water and ice to the sulphomaleate and reduction of the pH to about 1, preferably at a temperature of from 25°C to 30°C, in the absence of alkali or alkaline earth metal ions, to retain the double bond and to form sulphomaleic acid;
(iii) addition of an appropriate diaminoalkyl moiety to the sulphomaleic acid together with alkali and/or alkaline earth metal hydroxides, suitably to about pH 11. The temperature is suitably maintained at from 25°C to 50°C, preferably from 25°C to 35°C, with agitation for from 2 to 5 hours.

The diaminoalkyl moiety may be ethylenediamine, 1,3-propylenediamine, 2-hydroxy-1,3-propylenediamine or 1,2-propylenediamine.

After formation, the material is suitably recovered by vacuum distillation. The process produces a good yield of DDSS.

Sulphur trioxide reacts with maleic anhydride to afford sulphomaleic anhydride (SMA) according to the following reaction scheme:

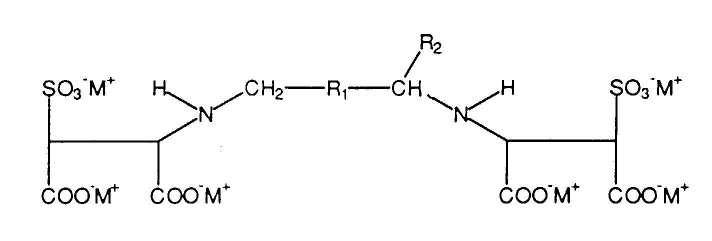

Because of the presence of the sulphonic acid group, sulphomaleic anhydride (SMA) is a much more reactive electrophile than maleic anhydride and will therefore react rapidly with nucleophiles under aqueous Michael conditions.

Diamine derivatives add to either the sodium or calcium salts of SMA.

When the starting Michael addend is a diamine, for example, an alkylenediamine such as ethylene diamine, then diaddition to sulphomaleate occurs as the following reaction scheme shows to produce ethylenediamine di(sulphosuccinate).

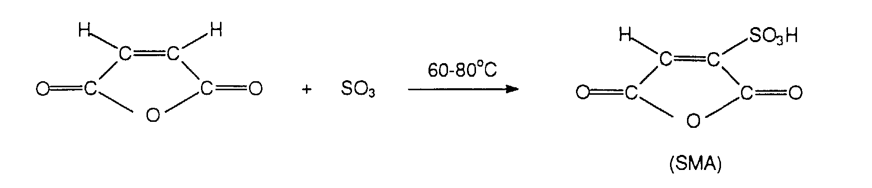

Ethylenediamine di(sulphosuccinate) (EDDSS) has eight ligands for binding which make this material a good builder. Sodium sulpho azadisuccinate (SADS), on the other hand, has six ligands.

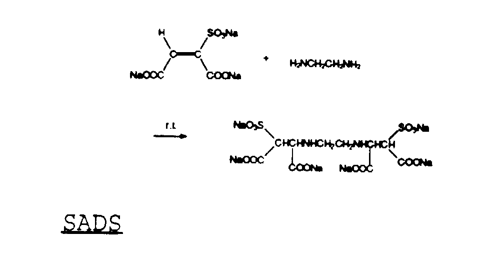

Amino based sulphosuccinates may be produced by reacting either an amine or or an amine carboxylate with the sulphomaleate at a pH of from 9 to 12 and at a temperature of about 25°C. Either calcium or sodium salts or mixtures of both can be employed, however, it is more convenient to use sodium because the amine builders appear to complex calcium strongly, and pose a removal problem. The addition, unlike the hydroxy acid route, produces a mixture of RS,SR,RR,SS isomers.

In general, the process involves running the reaction at a sufficient temperature for a sufficient time to form the final product while maintaining the following parameters.

The alkali or alkaline earth metal hydroxide or mixtures employed in the reaction mixtures of the inventive process is selected from the group consisting of sodium, potassium, lithium or barium hydroxide, or strontium hydroxide, or magnesium, or calcium hydroxide or mixtures of these. The most preferred alkali metal hydroxide for use in this invention is sodium hydroxide.

The DDSS salt forming reaction of the present invention is conducted at high concentration in aqueous media to afford efficacy and high throughput. The amount of water present may vary and is preferably sufficient to permit the reaction to proceed with the amount of water being about 55% to 95%. The amount may, however, be more or less depending on design parameters.

Desirably, the reactants of the starting mixture for the process are combined in water using physical agitation. In the preferred embodiments of the invention, the alkali or alkaline earth metal hydroxide or mixture is mixed with an aqueous mixture of the alkylenediamine and sulphomaleate moieties with agitation. The reaction is conducted at atmospheric pressure.

The reaction temperature for the process is preferably room temperature, 25°C or cooler. The reaction temperature is maintained for at least about 2 hours and preferably no longer than about 5 hours at temperatures ranging from 20°C to 35°C. The aqueous reaction product typically contains a mixture of DDSS, alkyldiamine and sulphomalate.

The reaction products obtained by the processes of this invention contain the alkali or alkaline earth metal or mixture of salts of DDSS and may be worked up by methods known in the art.

At any stage after the DDSS salt formation, the reaction product can be concentrated by removal of water to the desired extent. Water removal can, for example, involve substantially complete drying of the reaction product mixture, eg by spray drying, so that the DDSS salt is recovered in solid, eg granular form. The sodium salt of DDSS in the form of an aqueous liquid may be utilised directly in the preparation of detergent compositions or laundry additive products of the types more fully described hereinafter.

### Calcium binding

The log K_{Ca} of RS,SR hexasodium ethylenediamine di(sulphosuccinate) was calculated to be 8 with an SC5 of 1.6.

### Detergent compositions

When converted into suitable form, the DDSS salts can be used as sequestering builders in a wide variety of detergent or laundry additive compositions.

Detergent compositions of the invention are built laundry detergents which comprise, as a detergency builder, from 0.1 to 75 wt% of a diaminoalkyl di(sulphosuccinate) compound as defined above.

Compositions of the invention may suitably comprise
(a) from 1 to 75 wt% of one or more detergent surfactants, selected from anionic surfactants, nonionic surfactants, zwitterionic surfactants, ampholytic surfactants, cationic surfactants, and mixtures thereof;
(b) from 5 to 80 wt% of one or more detergency builders, wherein the composition comprises as a detergency builder (b) from 0.1 to 75 wt% of a diaminoalkyl di(sulphosuccinate) (DDSS) compound as defined previously, in alkali metal, alkaline earth metal, ammonium or substituted ammonium salt form, or mixtures thereof.

The DDSS may be present as a supplement to another, primary builder. Compositions of this embodiment may comprise:
(a) from 1 to 75 wt% of a detergent surfactant,
(b) from 5 to 80 wt% of a primary detergency builder; and
(c) from 0.1% to 75 wt% of DDSS as a secondary builder.

Alternatively, the DDSS can, if desired, be used as the sole builder.

Surfactants that are useful in the present invention are the anionic (soap and nonsoap), nonionic, zwitterionic and ampholytic compounds. The chemical nature of these detergent compounds is not an essential feature of the present invention. Moreover, such detergent compounds are well known to those skilled in the detergent art and the patent and printed literature are replete with disclosures of such compounds. Typical of such literature are "Surface Active Agents" by Schwartz and Perry and Berch, the disclosures of which are incorporated by reference herein.

As indicated, the DDSS builder can be used either as the sole builder or where desired can be used in conjunction with other well-known builders, examples of which include water-soluble salts of phosphates, pyrophosphates, orthophosphates, polyphosphates, phosphonates, carbonates, polyhydroxy-sulphonates, polyacetates, carboxylates, polycarboxylates, succinates and the like; and water-insoluble builders such as alkali metal aluminosilicates (zeolites).

In addition to the surfactant and builder, there may be optionally present additional ingredients which enhance the performance of the detergent composition. Typical examples thereof include the well known soil suspending agents, hydrotropes, corrosion inhibitors, dyes, perfumes fillers, optical brighteners, enzymes, suds boosters, suds depressants, germicides, anti-tarnishing agents, cationic detergents, softeners, bleaches, buffers and the like.

The detergent compositions of the present invention may be in any of the usual physical forms for such compositions, such as powders, beads, flakes, bars, tablets, noodles, liquids, pastes and the like.

The detergent compositions may be prepared and utilised in a conventional manner. The wash solutions thereof desirably have a pH from 7 to 12, preferably from 9 to 11.

In addition to their utility as builders in detergent and laundry additive compositions, the DDSS salts of the invention can also be utilised in other contexts wherein water hardness sequestration is required. Other uses are provided in water softening compositions, devices and methods and boiler descaling compositions and methods. It is also theorised that DDSS can complex heavy metals which react with bleach and thus can stabilise bleach.

It should also be noted that DDSS has utility in metal cleaning compositions under pH conditions of about 2 to about 5.

### EXAMPLES

The invention is illustrated by the following Examples. All percentages and parts herein are by weight unless indicated otherwise. All ratios herein are mole ratios unless indicated otherwise.

Compounds were characterised by NMR using a 200 MHz Bruker model. Samples were prepared by dissolution in D₂O.

### EXAMPLE 1

0.1 mole of sulphomaleic anhydride is dissolved in about 50 ml of water and stirred. 0.3 mole of sodium bicarbonate is then added to a pH of about 9 and the solution is cooled to maintain it at about 25°C (room temperature). 0.05 mole of ethylenediamine is added with continued agitation and the pH is maintained at 11 by addition of sodium hydroxide if necessary. The temperature is still maintained at 25°C. After five hours of reaction time, the solution is evaporated to dryness on a rotoval and dried in an oven. The material obtained is hexasodium ethylenediamine di(sulphosuccinate).
¹H NMR
   CH₂N multiplet 2.5 - 2.7 δ
   CHN multiplet 3.4 - 3.6 δ
   CHSO₃ multiplet 3.75 - 3.84 δ
C¹³ NMR
   CH₂N 4 singlets 51-53 ppm
   CHN 4 singlets 67-69 ppm
   CHSO₃ 3 singlets 74-76 ppm
   COONa 4 singlets 176-184 ppm

### EXAMPLE 2

0.1 mole of sulphomaleic anhydride is dissolved in about 50 ml of water and stirred. 0.3 mole of sodium bicarbonate is then added to a pH of about 9 and the solution is cooled to maintain it at about 25°C (room temperature). 0.05 mole of 2 hydroxy 1,3 propylenediamine is added with continued agitation and the pH is maintained at 11 by addition of sodium hydroxide if necessary. The temperature is still maintained at 25°C. After five hours of reaction time, the solution is evaporated to dryness on a rotoval and dried in an oven. The material obtained is hexasodium 2-hydroxy1,3-propylenediamine di(sulphosuccinate).
¹H NMR
   CH₂N multiplet 2.2 - 2.6 δ
   CHN multiplet 3.4 - 3.6 δ
   CHOH multiplet 3.4 - 3.6 δ
   CHSO₃ multiplet 3.75 - 3.84 δ
C¹³ NMR
   CH₂N 4 singlets 55.5 - 57 ppm
   CHN 4 singlets 68 - 70 ppm
   CHOH 4 singlets 72.5 - 75 ppm
   CHSO₃ 4 singlets 75.5 - 76 ppm
   COONa 5 singlets 176 - 184 ppm

### EXAMPLE 3

0.1 mole of sulphomaleic anhydride is dissolved in about 50 ml of water and stirred. 0.3 mole of sodium bicarbonate is then added to a pH of about 9 and the solution is cooled to maintain it at about 25°C (room temperature). 0.05 mole of 1,3 propylenediamine is added with continued agitation and the pH is maintained at 11 by addition of sodium hydroxide if necessary. The temperature is still maintained at 25°C. After five hours of reaction time, the solution is evaporated to dryness on a rotoval and dried in an oven. The material obtained is hexasodium 1,3- propylenediamine di(sulphosuccinate).
¹H NMR
   CH₂ multiplet 1.3 - 1.7 δ
   CH₂N multiplet 2.4 - 2.8 δ
   CHN doublet of doublets 3.4 - 3.6 δ
   CHSO₃ doublet of doublets 3.6 - 3.8 δ
C¹³ NMR
   CH₂ 4 singlets 37.5 - 39 ppm
   CH₂N 2 singlets 50 ppm
   CHN 2 singlets 68 - 70 ppm
   CHSO₃ 2 singlets 75 - 76 ppm
   COONa 5 singlets 173 - 184 ppm

### EXAMPLE 4

An aqueous crutcher slurry containing 46% by weight of water is spray-dried in a counter-current spray-drying tower to a base powder having a bulk density of 710 g/liter and a moisture content of 15.8%. The formulation of the powder prepared, in parts by weight, is as follows:

| | parts |
|---|---|
| C₁₂-C₁₅ alcohol 7EO ethoxylate | 3.0 |
| DDSS | 23.0 |
| Sodium carbonate | 5.0 |
| Sodium silicate | 6.0 |
| Water and minor components | 10.0 |

A mixture of anionic and nonionic surfactants containing 3.8 parts of C₁₀₋₁₃ alkylbenzene sulphonic acid and parts of a C₁₂₋₁₅ primary alcohol 7EO ethoxylate prepared by neutralizing the sulphonic acid with caustic soda solution of 50% by weight is prepared. This mixture is then heated under vacuum until the water content is reduced to about 3%, resulting in a mole ratio of water to anionic of 1.6. This mixture is then sprayed onto the powder.

A liquid mixture of sodium monostearyl phosphate and petroleum jelly in a weight ratio of 1.3:1 is then sprayed onto the powder at the rate of 0.8 parts to 63 parts.

Finally, the powder is dosed with heat-sensitive components such as oxygen bleaches, perfumes and enzymes in accordance with conventional practice to produce a finished powder having the following composition (weight percent) and having a bulk density of about 800 g/litre:

| | |
|---|---|
| | |
| Sodium C₁₀₋₁₃ alkylbenzene sulphonate | 4.0 |
| C₁₂₋₁₅ primary alcohol ethoxylate 7EO | 9.0 |
| Hexasodium ethylenediamine di(sulphosuccinate) | 23.0 |
| Sodium carbonate | 5.0 |
| Sodium silicate | 6.0 |
| Sodium sulphate | 26.9 |
| Sodium perborate | 12.0 |
| Sodium carboxymethylcellulose | 0.9 |
| Sodium stearyl phosphate | 0.2 |
| Petroleum jelly | 0.6 |
| Enzyme marumes | 0.4 |
| Cellulose ether anti-redeposition aid | 0.3 |
| Water, perfume, and minor components to | 100.0 |

## Claims

1. A diaminoalkyl di(sulphosuccinate) compound of the formula I 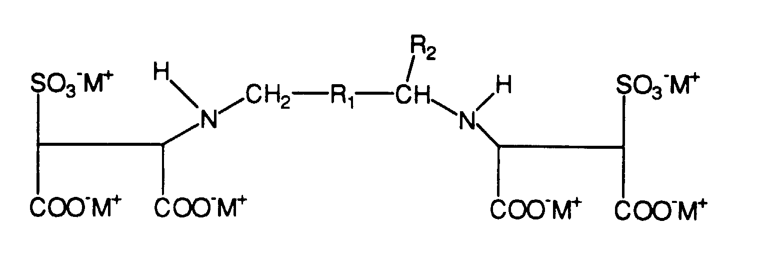 wherein
M is an alkali metal or an alkaline earth metal or a mixture; and
R₁ is CH₂ or CHOH and is optionally present, and when R₁ is present R₂ is hydrogen, and when R₁ is absent R₂ is methyl or hydrogen.

2. A compound as claimed in claim 1, wherein R₁ is absent and M is sodium.

3. A compound as claimed in claim 1, wherein R₁ is absent, R₂ is methyl and M is sodium.

4. A compound as claimed in claim 1, wherein R₁ is CH₂ and M is sodium.

5. A compound as claimed in claim 1, wherein R₁ is CHOH and M is sodium.

6. A process for the preparation of a compound as claimed in claim 1, which comprises:
(i) reacting sulphur trioxide with maleic anhydride to form sulphomaleic anhydride,
(ii) converting the sulphomaleic anhydride thus formed to sulphomaleic acid with reaction with water in the absence of alkali metal or alkaline earth metal ions,
(iii) reacting the sulphomaleic acid with a diamine selected from ethylene diamine, 1,3-propylene diamine,2-hydroxy-1,3-propylene diamine and 1,2-propylene diamine in the presence of alkali and/or alkaline earth metal hydroxide.

7. A method for chelating divalent or polyvalent metal ions, which comprises contacting said ions with a diaminoalkyl di(sulphosuccinate) as claimed in any one of claims 1 to 5.

8. A built detergent composition which comprises one or more detergent surfactants and, as a detergency builder, from 0.1 to 75 wt% of a diaminoalkyl di(sulphosuccinate) compound as claimed in any one of claims 1 to 5.

9. A built detergent composition as claimed in claim 8, which comprises:
(a) from 2 to 98 wt% of one or more detergent surfactants,
(b) from 5 to 80 wt% of one or more detergency builders,
wherein the composition comprises as a detergency builder (b) from 0.1 to 75 wt% of a diaminoalkyl di(sulphosuccinate) compound as claimed in any one of claims 1 to 5.

## Patentansprüche

1. Eine Diaminoalkyldi(sulfosuccinat)-Verbindung der Formel I 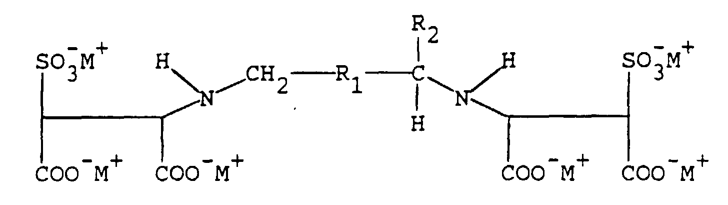 worin
M ein Alkalimetall oder ein Erdalkalimetall oder eine Mischung ist; und
R₁ CH₂ oder CHOH bedeutet und wahlweise vorhanden ist, und, wenn R₁ anwesend ist, ist R₂ Wasserstoff, und wenn R₁ fehlt, ist R₂ Methyl oder Wasserstoff.

2. Eine Verbindung, wie in Anspruch 1 beansprucht, worin R₁ fehlt und M Natrium ist.

3. Eine Verbindung, wie in Anspruch 1 beansprucht, worin R₁ fehlt, R₂ Methyl ist und M Natrium bedeutet.

4. Eine Verbindung, wie in Anspruch 1 beansprucht, worin R₁ CH₂ ist und M Natrium bedeutet.

5. Eine Verbindung, wie in Anspruch 1 beansprucht, worin R₁ CHOH ist und M Natrium bedeutet.

6. Ein Verfahren für die Herstellung einer Verbindung, wie in Anspruch 1 beansprucht, welches umfaßt:
(i) Umsetzen von Schwefeltrioxid mit Maleinsäureanhydrid zur Bildung von Sulfomaleinsäureanhydrid,
(ii) Umwandeln des so gebildeten Sulfomaleinsäureanhydrids zu Sulfomaleinsäure durch Reaktion mit Wasser in Abwesenheit von Alkalimetall- oder Erdalkalimetall-Ionen,
(iii) Umsetzen der Sulfomaleinsäure mit einem Diamin, ausgewählt aus Ethylendiamin, 1,3-Propylendiamin, 2-Hydroxy-1,3-propylendiamin und 1,2-Propylendiamin in Gegenwart von Alkali- und/oder Erdalkalimetallhydroxid.

7. Ein Verfahren für chelatbildende divalente oder polyvalente Metallionen, welches die Kontaktbehandlung der erwähnten Ionen mit einem Diaminoalkyldi(sulfosuccinat), wie in irgendeinem der Ansprüche 1 bis 5 beansprucht, umfaßt.

8. Eine aufgebaute Waschmittelzusammensetzung, welche ein oder mehrere Waschmittel-Surfactants, und, als einen Waschkraftbuilder von 0,1 bis 75 Gewichtsprozent einer Diaminoalkyldi(sulfosuccinat)-Verbindung, wie in irgendeinem der Ansprüche 1 bis 5 beansprucht, enthält.

9. Eine aufgebaute Waschmittelzusammensetzung, wie in Anspruch 8 beansprucht, welche umfaßt:
(a) Von 2 bis 98 Gewichtsprozent von einem oder mehreren Waschmittel-Surfactants,
(b) von 5 bis 80 Gewichtsprozent von einem oder mehreren Waschkraftbuildern,
worin die Zusammensetzung als einen Waschkraftbuilder (b) von 0,1 bis 75 Gewichtsprozent einer Diaminoalkyldi(sulfosuccinat)-Verbindung, wie in irgendeinem der Ansprüche 1 bis 5 beansprucht, enthält.

## Revendications

1. Composé diaminoalkyl-di(sulfosuccinate) de formule (I) : 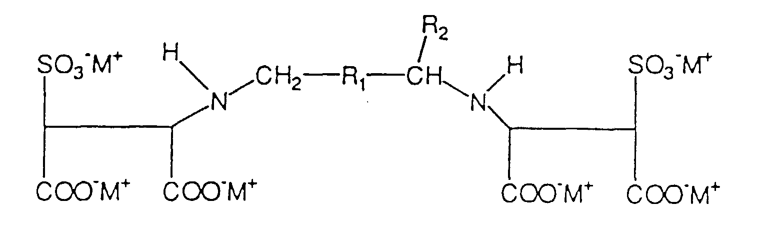 dans laquelle
M est un métal alcalin ou un métal alcalino-terreux ou un mélange ; et
R₁ est CH₂ ou CHOH et est facultativement présent et quand R₁ est présent, R₂ est l'hydrogène et quand R₁ est absent, R2 est le méthyle ou l'hydrogène.

2. Composé selon la revendication 1, dans lequel R₁ est absent et M est le sodium.

3. Composé selon la revendication 1, dans lequel R₁ est absent, R₂ est le méthyle et M est le sodium.

4. Composé selon la revendication 1, dans lequel R₁ est CH₂ et M est le sodium.

5. Composé selon la revendication 1, dans lequel R₁ est CHOH et M est le sodium.

6. Procédé de préparation d'un composé selon la revendication 1, qui comprend :
(i) la réaction d'anhydride sulfurique avec de l'anhydride maléique pour former un anhydride sulfomaléique,
(ii) la conversion de l'anhydride sulfomaléique ainsi formé en acide sulfomaléique avec réaction avec l'eau en l'absence d'ions de métaux alcalins ou de métaux alcalino-terreux,
(iii) la réaction de l'acide sulfomaléique avec une diamine choisie parmi l'éthylènediamine, la 1,3-propylènediamine la 2-hydroxy-1,3-propylènediamine et la 1,2-propylènediamine en présence d'hydroxyde de métal alcalin et/ou de métal alcalino-terreux.

7. Procédé de chélation d'ions métalliques divalents ou polyvalents qui comprend la mise en contact desdits ions avec un diaminoalkyl-di(sulfosuccinate) selon l'une quelconque des revendications 1 à 5.

8. Composition détergente avec adjuvant qui comprend un ou plusieurs tensioactifs détergents et, à titre d'adjuvant de détergence, 0,1 à 75% en poids d'un composé diaminoalkyl-di(sulfosuccinate) selon l'une quelconque des revendications 1 à 5.

9. Composition détergente avec adjuvant selon l'une quelconque des revendications 8, qui comprend :
(a) de 2 à 98% en poids d'un ou plusieurs tensioactifs détergents,
(b) de 5 à 80% en poids d'un ou plusieurs adjuvants de détergence,
la composition comprenant à titre d'adjuvant de détergence (b) de 0,1 à 75% en poids d'un composé diaminoalkyl-di(sulfosuccinate) selon l'une quelconque des revendications 1 à 5.
